# EUROPEAN PATENT SPECIFICATION

(11) **EP 2 808 680 B1**
(45) Date of publication and mention of the grant of the patent: **19.08.2020**
(21) Application number: 12866956.1
(22) Date of filing: 20.03.2012
(51) Int. Cl.: G01N 33/497, G01N 33/98

(54) **CARTRIDGE PROVIDED WITH STANDARD ALCOHOL GAS FOR BREATHALYZER**
KASSETTE MIT EINEM STANDARDALKOHOLGAS FÜR EIN ALKOHOLTESTGERÄT
CARTOUCHE POUR ÉTHYLOTEST RENFERMANT UN ALCOOL STANDARD SOUS FORME GAZEUSE

(30) Priority: 26.01.2012 KR 20120007490
(43) Date of publication of application: 03.12.2014
(73) Proprietor: Elechem Co., Ltd., Incheon 403-030 (KR)
(72) Inventor: PARK, Kwang Hee, Seoul 134-060 (KR); PARK, Ik Hyun, Incheon 402-040 (KR)
(74) Representative: Sloboshanin, Sergej
(86) International application number: PCT/KR2012/001991
(87) International publication number: WO 2013/111926

(56) References cited:
- WO-A2-97/14947
- KR-B1- 100 687 787
- KR-B1- 100 827 714
- KR-B1- 101 059 978
- US-A- 5 400 637
- US-A1- 2009 056 408

## Description

### Technical Field

The present invention relates to a standard alcohol gas cartridge for a drunkometer, and more particularly to a standard alcohol gas cartridge for a drunkometer being capable of correcting the measurement error easily and quickly to achieve an accurate measurement and high reliability.

### Background Art

Usually, a drunkometer comprises an alcohol sensor, a signal processor and a display. The alcohol sensor reacts with the alcohol in the exhaled breath and outputs the electrical energy, which is processed and calculated into the alcoholic concentration in the signal processor, and the calculated alcoholic concentration is displayed in the display. But the drunkometer may incur a measurement error inevitably due to the change of the electrochemical characteristics of the alcohol sensor depending on the service environment and service life, which would result in a reduced reliability of the measurement of the alcoholic concentration, and if the personal drunkometer has this problem, then it will incur disputes between the driver and the police officer owing to discrepancy of the measured results of the personal drunkometer and the police officer's drunkometer.

Therefore, a periodic test is required to determine whether the drunkometer operates accurately, and if the drunkometer is not accurate, then correction of the drunkometer should be implemented. In order to test and correct the drunkometer, the test method to use standard alcohol with the predetermined concentration is generally adopted. But this method to use standard alcohol generally requires expensive technical equipment and safety apparatus, and the test or correction methods are different depending on the manufactures of the drunkometer. As a result, the test or correction of the drunkometer is usually implemented by skilled personnel. Therefore, the test and correction of the drunkometer requires high cost, long time and much effort, so the drunkometer may not be tested and corrected timely, and the inaccurate drunkometer causes troubles between the driver and the police officer.

Document WO 97/14947 discloses devices for providing vapour samples of volatile components of a solution and apparatus for allowing such samples to be tested. Two different sample containers are described. In each, a volatile solution is retained so that the volatile component equilibrates with a headspace from whence it can be drawn off into testing apparatus. In the first device, the volatile component is retained by a gas-permeable-hydrophobic membrane (14), whilst in the second device the solution is retained in absorbent material. The first device has associated apparatus, whilst the second device is complete in itself. In each case, when the testing apparatus is located on a sampling outlet port, it causes a valve to open allowing sampling to occur.

### Disclosure

### Technical Problem

In order to solve the above-mentioned problems of the conventional art, it is an object of the present invention to provide a standard alcohol gas cartridge for a drunkometer being capable of correcting a measurement error easily and quickly to achieve an accurate measurement and high reliability of the drunkometer and save the cost and time associated with the correction of the drunkometer without assistance of technical equipment and skilled personnel.

### Technical Solution

To attain the above object of the present invention, according to the present invention, there is provided a standard alcohol gas cartridge for a drunkometer which contains the standard alcohol gas with the predetermined concentration, the value of the predetermined concentration of the standard alcohol gas being displayed on the outer surface of the cartridge 1 or is stored on the storing medium provided on the cartridge 1, the cartridge 1 having a gas outlet port 13 which is connectable to the drunkometer 30 to exhaust the standard alcohol gas to the drunkometer 30.

The cartridge 1 comprises further an inner container 20 of soft material in which the standard alcohol gas with the predetermined concentration is contained and an outer case 10 of hard material to protect the inner container 20. The gas outlet port 13 is sealed by a soft membrane 16 configured to be penetrated by a gas inlet pin 51 of the drunkometer 30 without leakage of the standard alcohol gas to achieve smooth flow of the standard alcohol gas. The cartridge 1 further includes an interface 14 which stores the information of the concentration value of the standard alcohol gas, transmits the information of the predetermined concentration of the standard alcohol gas to the drunkometer 30 and detects whether the cartridge 1 is connected to the drunkometer 30.

According to an embodiment, the interface 14 on the side of the cartridge 1 comprises further a press projection 15, and the drunkometer 30 includes touch switches 44, 45 to be pressed by the press projection 15 of the cartridge 1.

### Advantageous Effects

According to the present invention, by providing the portable standard alcohol gas cartridge 1 which contains the standard alcohol gas with the predetermined concentration, and the value of the concentration of the standard alcohol gas being displayed on the outer surface of the cartridge 1 or being stored on the storing medium provided in the cartridge 1, and the cartridge 1 being connectable to the drunkometer 30 to exhaust the standard alcohol gas to the drunkometer 30, the drunkometer 30 can be easily tested and corrected by connecting this standard gas cartridge 1 to the drunkometer 30 without assistance of technical equipment and skilled personnel and can reduce the cost and time associated with the drunkometer test and correction. And as the drunkometer can be tested and corrected timely, the accuracy and reliability of the drunkometer can be enhanced or kept highly, so the drunken driving would be effectively avoided or prevented.

And, as the portable standard gas cartridge 1 further includes the interface 14, which can transmit the signal of detecting the drunkometer 30 being connected to the portable standard gas cartridge 1 and the information of the predetermined alcohol concentration of the standard alcohol gas contained in the cartridge 1, it would not be necessary to manually input the information of the alcoholic concentration of the standard gas into the drunkometer 30, so the operating process of the test and correction is easy and quick.

And as the interface 14 is made of a press projection 15 and the position of the press projection 15 is to be changed according to the concentration value of the standard gas, and the drunkometer 30 further includes touch switches 45, 44 to be pressed by the press projection 15, then the different contact position or pattern of the press projection 15 against the touch switches 44, 45 will specify the different concentration value of the standard alcohol gas of the cartridge 1, so that the information of the alcohol concentration of the standard gas cartridge 1 could be easily transmitted to the drunkometer 30 without use of expensive equipment.

Moreover, as the cartridge 1 comprises an inner container 20 of soft material and an outer case 10 of hard material protecting the inner container 20, the standard alcohol gas contained in the inner container 20 will be effectively sealed, and the phenomenon of forced inflow of the standard gas into the alcohol sensor 50 by the outer pressure and the resulted measurement error will be effectively prevented to achieve a precise and accurate correction. And the gas outlet port 13 of the portable standard gas cartridges 1 is sealed by a soft membrane 16, and the gas inlet pin 51 of the drunkometer 30 can be easily inserted into the gas outlet port 13 of the standard gas cartridge 1 without leakage of the standard gas to achieve smooth flow of the standard gas.

### Description of the Drawings

The above and other objects and advantages of the invention will become more apparent by describing a preferred embodiment with reference to the accompanying drawings in which:

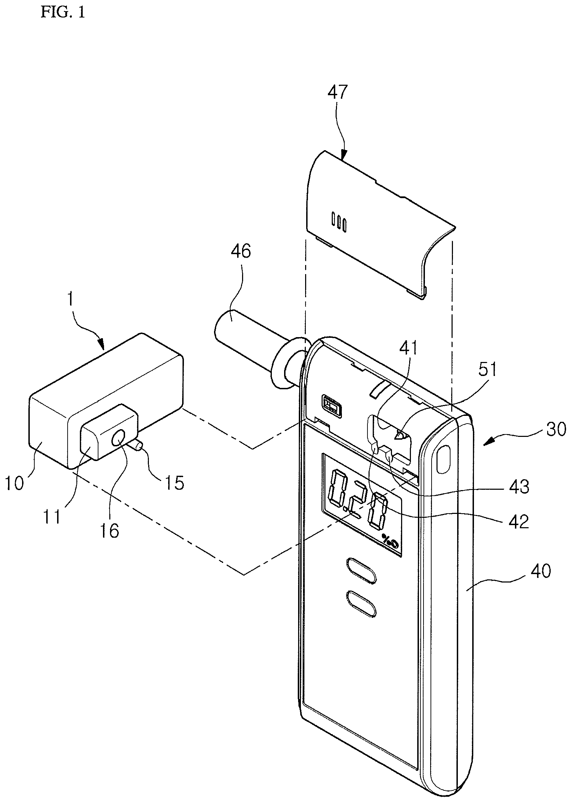

FIG. 1 is an explosive view illustrating use state of an embodiment of the present invention;

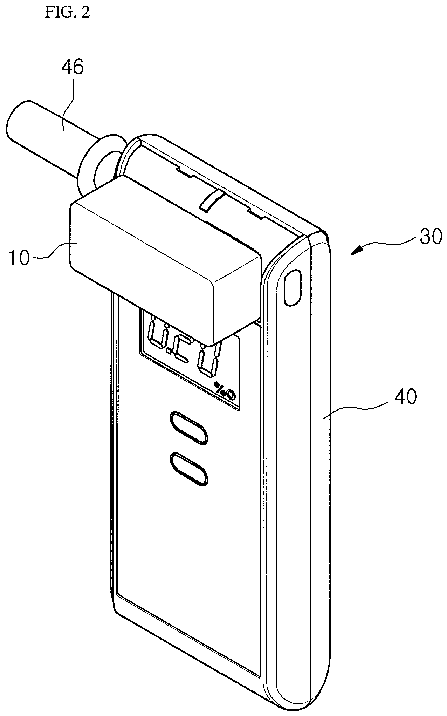

FIG. 2 is perspective view illustrating the assembled state of above embodiment of the invention

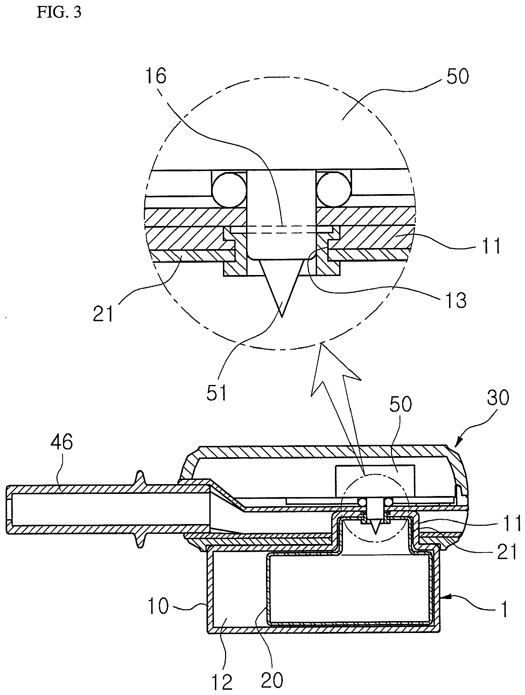

FIG. 3 is a first sectional plan of FIG. 2

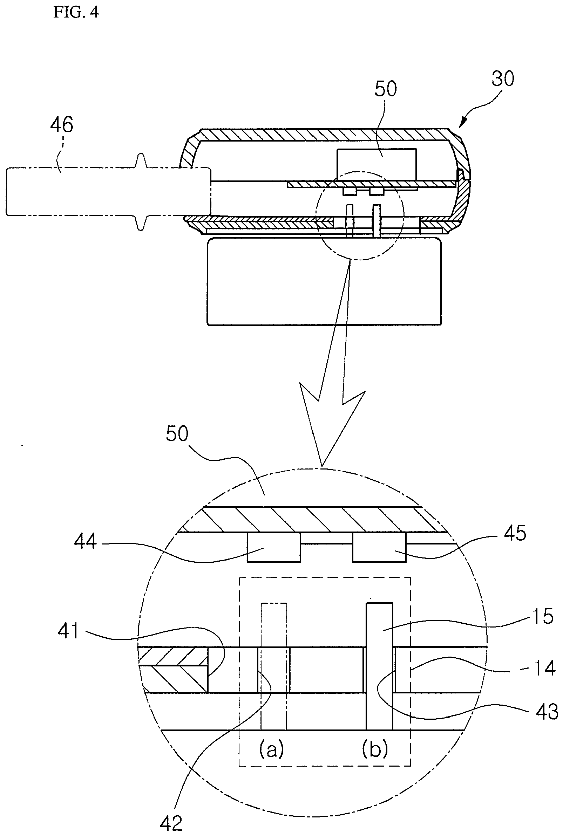

FIG. 4 is a second sectional plan of FIG. 2

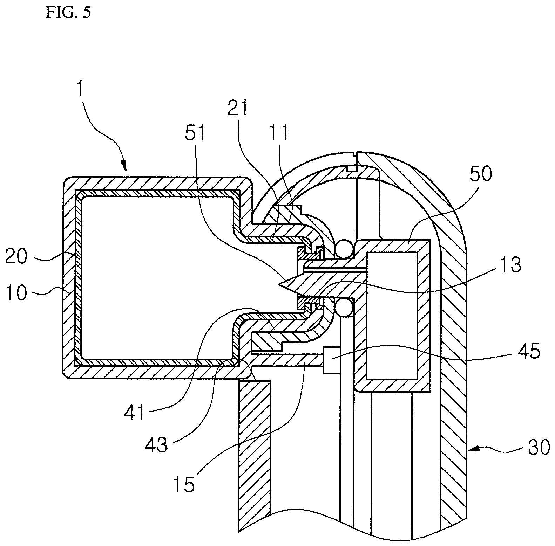

FIG. 5 is a side section of FIG. 2

### Detailed Description of the Invention

Hereinbelow, a standard alcohol gas cartridge for a drunkometer according to a preferred embodiment of the present invention will be described with reference to the accompanying drawings.

A standard alcohol gas cartridge 1 of the present invention includes a portable outer case 10 connectable to the drunkometer 30, an inner container 20 provided in the outer case 10 and containing the standard alcohol gas with the predetermined concentration, and an interface 14 to transmit the information of the concentration of the standard alcohol gas to the drunkometer 30.

The outer case 10 is formed in cubic shape, and an inserting projection 11 is formed on one side of it. A hollow part 12 is formed inside the outer case 10, which extends to the inserting projection 11. The outer case 10 is preferably made of hard material to prevent the shape of the hollow part 12 from being deformed or to prevent the inner pressure of the hollow part 12 from being changed. For example, the outer case 10 would be made of hard material such as metal, polypropylene, ABS or polycarbonate, As a result, the inner container 20 may be prevented from being damaged by outer force.

The inner container 20 contains the standard alcohol gas and is disposed in the hollow part 12 of the outer case 10. The inner container 20 is made of soft plastic membrane to seal the standard gas from leakage and keep it from evaporating. The charge amount of the standard alcohol gas in the cartridge 1 is properly adjusted to avoid the damage of the inner container 20 owing to the inflation of the charged standard alcohol gas. An extended projecting 21 is provided on one side of the inner container 20 and it is inserted into the inserting projection 11 of the outer case 10. And a gas outlet port 13 is formed to penetrate to the extended projection 21 of the inner container 20 and the inserting projection 11 of the outer case 10, and this gas outlet port 13 is sealed by s soft membrane 16.

The interface 14 stores the information of the concentration value of the standard alcohol gas, transmits it to the drunkometer 30, and detects whether the cartridge 1 is connected to the drunkometer 30 or not. The interface 14 of the illustrated embodiment comprises a press projection 15 being adjacent to the inserting projection 11 to press the press switches of the drunkometer 30. Alternatively, the information of the concentration value of the standard alcohol contained in the inner container 20 could be stored in a storing medium provided on the outer case 10, and could be transmitted to the drunkometer 30 through the interface 14. Meanwhile, the method for the interface 14 to store and transmit the information of the concentration of the standard alcohol gas to the drunkometer 30 may be RFID, NFC or USB alternatively.

The position of the press projection 15 of the outer case 10 is determined differently depending on the concentration values of the standard alcohol gases. For example, the press projection 15 of the standard gas cartridge 1 containing a standard alcohol gas of 0.05%BAC is positioned on (a), and the press projection 15 of the standard gas cartridge 1 containing a standard alcohol gas of 0.1%BAC is positioned on (b). A plurality of press projections 15 can be provided on the outer case 10 and the combination of a plurality of the positions of the press projections 1 can represent the predetermined concentration of the standard alcohol gas.

The standard gas cartridge 1 according to the present invention is mounted on the drunkometer 30, in which the exhaled breath induced through the exhale tube 46 by the pump driven by an unseen solenoid or driving motor flows into the alcohol sensor 50 and the alcohol concentration is calculated. The drunkometer 47 includes a cover 47 on one side of the upper end of the main body 40. On the upper part of the main body 4 an inserting window 41 is formed, into which the inserting projection 11 of the cartridge 1 is inserted, and on one side of the insert window 41 a pair of punching holes 42, 43 is provided corresponding to the positions (a) and (b) of the press projection 15 of the standard gas cartridge 1. A gas inlet pin 51 is provided in the inserting window 41 and it is communicated with the alcohol sensor 50. This gas inlet pin 51 is connectable to the gas outlet port 13 of the cartridge 1, and it can easily penetrate into the soft membrane 16 covering the gas outlet port 13 of the cartridge 1.

The main body 40 of the drunkometer 30 includes a first and a second touch switch 44, 45 which could be pressed by the press projection 15 positioned on (a) or (b) of the cartridge 1 when the gas outlet port 13 is connected to the gas inlet pin 51 of the main body 40 of the drunkometer 1. As a result, it could be detected that a standard gas cartridge 1 of a certain value of alcoholic concentration is mounted on the main body 40 of the drunkometer 30.

In order to test whether the drunkometer 30 works properly, the user should connect the standard gas cartridge 1 into the inserting window 41 of the main body 40 of the drunkometer 30, then the standard gas in the cartridge 1 will flow into the alcohol senor 50 of the drunkometer 30. At this time, the fact that the cartridge 1 is connected to the drunkometer 30 and the information of the concentration of the standard gas alcohol is transmitted to the drunkometer 30 through the interface 14. Then the drunkometer 30 measures the concentration of the standard alcohol gas and compares the actually measured value with the predetermined value of concentration of the standard gas alcohol. If the measured value of the standard gas coincides with that of the predetermined concentration of the standard gas, that is, the difference is within the allowable error range, it is determined that the drunkometer works properly and there is no need of correction or calibration. If the measured value of the standard gas does not coincide with that of the predetermined concentration of the standard gas, that is, the difference is not within the allowable error range, then the user implements the correction or calibration action until the measured value coincides with that of the predetermined concentration of the standard gas.

## Claims

1. A portable standard alcohol gas cartridge (1) for a drunkometer (30) which contains a standard alcohol gas with a predetermined concentration, the value of the predetermined concentration of the standard alcohol gas being displayed on the outer surface of the cartridge (1) or is stored on the storing medium provided on the cartridge (1), the cartridge (1) having a gas outlet port (13) which is connectable to the drunkometer (30) to exhaust the standard alcohol gas to the drunkometer (30)
**characterized in that**
the cartridge (1) comprises an inner container (20) of soft material in which the standard alcohol gas with the predetermined concentration is contained and an outer case (10) of hard material to protect the inner container (20), and the gas outlet port (13) is sealed by a soft membrane (16) configured to be penetrated by a gas inlet pin (51) of the drunkometer (30) without leakage of the standard alcohol gas to achieve smooth flow of the standard alcohol gas,
wherein the cartridge (1) further includes an interface (14) which stores the information of the concentration value of the standard alcohol gas, transmits the information of the predetermined concentration of the standard alcohol gas to the drunkometer (30) and detects whether the cartridge (1) is connected to the drunkometer (30).

2. Portable standard alcohol gas cartridge (1) for a drunkometer (30) according to claim 1, wherein the interface (14) on the side of the cartridge (1) comprises a press projection (15), and the drunkometer (30) includes touch switches (44), (45) to be pressed by the press projection (15) of the cartridge (1).

## Patentansprüche

1. Tragbare Standardalkoholgaskartusche (1) für eine Alkoholtestvorrichtung (30), die ein Standardalkoholgas mit einer vorherbestimmten Konzentration enthält, wobei der Wert der vorherbestimmten Konzentration des Standardalkoholgases auf der Außenoberfläche der Kartusche (1) angezeigt ist oder in dem auf der Kartusche (1) vorgesehenen Speichermedium gespeichert ist, wobei die Kartusche (1) eine Gasauslassöffnung (13) aufweist, die mit der Alkoholtestvorrichtung (30) verbindbar ist, um das Standardalkoholgas an die Alkoholtestvorrichtung (30) abzugeben,
**dadurch gekennzeichnet, dass**
die Kartusche (1) einen Innenbehälter (20) aus weichem Material, in dem das Standardalkoholgas mit der vorherbestimmten Konzentration enthalten ist, und ein Außengehäuse (10) aus hartem Material umfasst, um den Innenbehälter (20) zu schützen, und die Gasauslassöffnung (13) durch eine weiche Membran (16) abgedichtet ist, die derart ausgelegt ist, dass sie von einem Gaseinlassstift (51) der Alkoholtestvorrichtung (30) ohne Leckage des Standardalkoholgases durchdrungen wird, um einen gleichmäßigen Strom des Standardalkoholgases zu erreichen,
wobei die Kartusche (1) ferner eine Schnittstelle (14) umfasst, die die Information über den Konzentrationswert des Standardalkoholgases speichert, die Information über die vorherbestimmte Konzentration des Standardalkoholgases an die Alkoholtestvorrichtung (30) überträgt und erfasst, ob die Kartusche (1) mit der Alkoholtestvorrichtung (30) verbunden ist.

2. Tragbare Standardalkoholgaskartusche (1) für eine Alkoholtestvorrichtung (30) nach Anspruch 1, wobei die Schnittstelle (14) auf der Seite der Kartusche (1) einen Druckvorsprung (15) umfasst, und die Alkoholtestvorrichtung (30) Berührungsschalter (44), (45) aufweist, die von dem Druckvorsprung (15) der Kartusche (1) gedrückt werden sollen.

## Revendications

1. Cartouche de gaz alcoolisé standard portable (1) pour un alcootest (30) qui contient un gaz alcoolisé standard à une concentration prédéterminée, la valeur de la concentration prédéterminée du gaz alcoolisé standard étant affichée sur la surface extérieure de la cartouche (1) ou stockée sur le support de stockage ménagé sur la cartouche (1), la cartouche (1) présentant un orifice de sortie de gaz (13) qui peut être raccordé à l'alcootest (30) afin de laisser s'échapper le gaz alcoolisé standard jusqu'à l'alcootest (30),
**caractérisée en ce que**
la cartouche (1) comprend un récipient interne (20) en matériau mou, dans lequel est contenu le gaz alcoolisé standard à la concentration prédéterminée et un boîtier externe (10) en matériau dur permettant de protéger le récipient interne (20), et l'orifice de sortie de gaz (13) est scellé par une membrane molle (16) configurée afin d'être pénétrée par une broche d'entrée de gaz (51) de l'alcootest (30) sans fuite du gaz alcoolisé standard afin d'obtenir un écoulement régulier du gaz alcoolisé standard,
dans laquelle la cartouche (1) inclut en outre une interface (14) qui stocke les informations de la valeur de concentration du gaz alcoolisé standard, transmet les informations de la concentration prédéterminée du gaz alcoolisé standard à l'alcootest (30) et détecte si la cartouche (1) est connectée à l'alcootest (30).

2. Cartouche de gaz alcoolisé standard portable (1) pour un alcootest (30) selon la revendication 1, dans laquelle l'interface (14) sur le côté de la cartouche (1) comprend une saillie de pression (15), et l'alcootest (30) inclut des commutateurs tactiles (44), (45) à enfoncer par la saillie de pression (15) de la cartouche (1).
